# EUROPÄISCHE PATENTSCHRIFT

(11) **EP 1 763 520 B1**
(45) Veröffentlichungstag und Bekanntmachung des Hinweises auf die Patenterteilung: **17.12.2008**
(21) Anmeldenummer: 05756337.1
(22) Anmeldetag: 30.06.2005
(51) Int. Cl.: C07D 311/18

(54) **VERWENDUNG VON TRISUBSTITUIERTEN BENZOPYRANONEN**
USE OF TRISUBSTITUTED BENZOPYRANONES
UTILISATION DE BENZOPYRANONES TRISUBSTITUEES

(30) Priorität: 05.07.2004 DE 102004032440
(43) Veröffentlichungstag der Anmeldung: 21.03.2007
(73) Patentinhaber: Dr. Willmar Schwabe GmbH & Co. KG, 76227 Karlsruhe (DE)
(72) Erfinder: GERMER, Stefan, 76227 Karlsruhe (DE); HAUER, Hermann, 76228 Karlsruhe (DE); KOCH, Egon, 76229 Karlsruhe (DE); SCHÖTZ, Karl, 76448 Durmersheim (DE)
(74) Vertreter: Adam, Holger
(86) Internationale Anmeldenummer: PCT/EP2005/007051
(87) Internationale Veröffentlichungsnummer: WO 2006/002918

(56) Entgegenhaltungen:
- US-A1- 2003 175 777
- LATTE, KLAUS PETER ET AL: "Unusual coumarin patterns of Pelargonium species forming the origin of the traditional herbal medicine umckaloabo" ZEITSCHRIFT FUER NATURFORSCHUNG, C: JOURNAL OF BIOSCIENCES , 55(7/8), 528-533 CODEN: ZNCBDA; ISSN: 0939-5075, 2000, XP009061282 in der Anmeldung erwähnt

## Beschreibung

Die Erfindung betrifft die Verwendung von trisubstituierten Benzopyranonen zur Behandlung oder Prophylaxe von Krankheitszuständen, die mit oxidativem Stress und/oder Entzündungsreaktionen einhergehen sowie neue trisubstituierte Benzopyranone, und deren physiologisch verträgliche Salze. Die Erfindung betrifft ferner diese Verbindungen enthaltende Pflanzenextrakte, Arzneimittel, diätetische Nahrungsmittel und pharmazeutische Zubereitungen.

Freie Radikale sind Atome oder Moleküle, die über ein ungepaartes Elektron im äußeren Orbit verfügen. Das für biologische Prozesse bedeutendste freie Radikal ist molekularer Sauerstoff, aus dem durch Reduktion unterschiedliche Metabolite geformt werden können, die allgemein unter dem Sammelbegriff reaktive Sauerstoffverbindungen (ROS) zusammengefasst werden. Zu den ROS werden z. B. Superoxidanion, Hydroxylradikal, Wasserstoffperoxid, Peroxidanion, Singulett-Sauerstoff, Hypochlorid, Stickstoffoxid und Peroxynitrit gezählt.

ROS werden spontan durch verschiedene biologische Prozesse gebildet. Von besonderer Bedeutung ist der sogenannte "Respiratory Burst" von Leukozyten, bei dem nach Stimulation der Zellen mit Mikroorganismen, Xenobiotika oder endogenen Stoffen durch Aktivierung einer membranständigen NADPH-Oxidase aus molekularem Sauerstoff das Superoxid-Radikal und andere ROS als Folgeprodukte entstehen. Der Respiratory Burst ist einer der wichtigsten Mechanismen der frühen, unspezifischen Immunabwehr und dient vor allem der Abtötung von eingedrungenen Infektionserregern und Tumorzellen. Daneben entstehen ROS vor allem durch Leckage von Elektronen aus unzureichend gekoppelten Reaktionen. Dies geschieht z. B. bei der Synthese von Prostaglandinen und Leukotrienen aus Arachidonsäure, während der mitochondrialen Atmung, unter ischämischen Bedingungen durch Xanthinoxidase katalysierte Oxidation von Hypoxanthin oder im Verlauf der Cytochrome P450-vermittelten Metabolisierung von Fremdstoffen.

Während der Respiratory Burst bei der Infektabwehr grundsätzlich eine erwünschte Reaktion darstellt, ist die erhöhte und anhaltende Bildung von ROS in der Regel nachteilig, da der oxidative Angriff nicht auf eindringende Mikroorganismen beschränkt ist sondern auch das körpereigene Gewebe deren toxischem Potential ausgesetzt ist. Dies gilt insbesondere bei nicht infektiösen Erkrankungen, wie z. B. der erhöhten Bildung von ROS im Verlauf von Autoimmunerkrankungen, bei degenerativen Erkrankungen, während einer Ischämie oder bei der Metabolisierung von Pharmaka. Die unerwünschten Effekte von freien Radikalen und ROS beruhen auf deren Wechselwirkung mit Nukleinsäuren (z. B. Induktion von DNA-Strangbrüchen), Proteinen (z. B. Denaturierung, Inaktivierung von Enzymsystemen), Kohlenhydraten (z. B. Depolymerisation von Hyaluronsäure) und vor allem Lipiden (z. B. Lipidperoxidation, Schädigung von Membranen, Bildung von proinflammatorischen Prostaglandinen und Leukotrienen).

Nachdem bereits vor ca. 50 Jahren die Beteiligung von reaktiven Sauerstoffverbindungen (ROS) an der Pathogenese verschiedener Erkrankungen postuliert wurde, gilt heute als gesichert, dass diese Moleküle eine wichtige Rolle bei der Pathogenese zahlreicher Erkrankungen spielen, wie z. B. Diabetes Mellitus Typ I und II, entzündlichen Erkrankungen (z. B. rheumatoide Arthritis, Asthma, Colitis ulzerosa, Psoriasis), bakteriellen und viralen Infektionen (z. B. Influenza, AIDS, virale Hepatitis), Atherosklerose, Ischämien, neurologischen Erkrankungen (z. B. Alzheimersche Krankheit, Morbus Parkinson und andere neurodegerative Erkrankungen), Katarakt, Sichelzellanämie und Tumorerkrankungen, darüberhinaus aber auch für Alterungsprozesse mitverantwortlich sind (A. Bendich (1994) in: B. Frei (Hrsg.) "Natural Antioxidants in Human Health and Disease", Academic Press, San Diego, S. 447; E. Peterhans (1997) J. Nutr. 127, 962 S; D. V. Parke (1999) in: T. K. Basu et al. (Hrsg.) "Antioxidants in Human Health", CAB International, S. 1).

Zum Schutz gegenüber den schädlichen Effekten von freien Radikalen und ROS besitzt der Organismus verschiedene Abwehrsysteme. Dazu zählen Vitamine (z. B. Vitamin E und C) und andere niedermolekulare Verbindungen (z. B. Glutathione, Harnsäure), antioxidative Enzyme (z. B. Superoxid-Dismutase, Catalase und Glutathion-Peroxidase) sowie metallbindende Proteine (z. B. Transferrin, Ceruloplasmin). Häufig sind die körpereigenen antioxidativen Systeme allerdings nur während der initialen Phase eines pathologischen Prozesses wirksam, da die mit fortlaufenden Krankheitsprozess gebildeten, erhöhten Konzentrationen von ROS die Kapazität der endogenen Schutzmechanismen weit übersteigen.

Unter oxidativem Stress versteht man deshalb ein Mißverhältnis zwischen der Konzentration von ROS und den antioxidativen Abwehrsystemen. Aufgrund der überragenden Bedeutung von ROS für zahlreiche Erkrankungen besteht deshalb ein außerordentliches Interesse an Substanzen mit antioxidativen Eigenschaften, die zur Prophylaxe und Therapie solcher Krankheitszustände eingesetzt werden können.

Da ROS von besonderer Bedeutung für Entzündungsreaktionen sind und oxidativer Stress häufig mit der erhöhten Synthese von proinflammatorischen Eicosanoiden (z. B. Prostaglandine, Leukotriene) und Zytokinen (z. B. IL-1, TNF-α, IL-6) einhergeht, besteht insbesondere ein Bedarf an Substanzen, die nicht nur über antioxidative Eigenschaften verfügen sondern zusätzlich auch die Bildung dieser Entzündungsmediatoren hemmen.

Aufgabe der vorliegenden Erfindung ist die Bereitstellung von Verbindungen zur Behandlung oder Prophylaxe von Krankheitszuständen, die mit oxidativem Stress und/oder Entzündungsreaktionen einhergehen.

Diese Aufgabe wird durch die Verwendung von Verbindungen der allgemeinen Formel I, 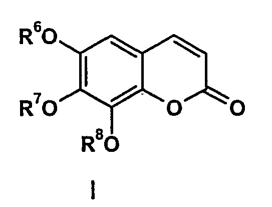 in der die Reste R⁶, R⁷ und R⁸ unabhängig voneinander H oder SO₃H bedeuten, sowie deren physiologisch verträglichen Salze zur Behandlung oder Prophylaxe von Krankheitszuständen, die mit oxidativem Stress und/oder Entzündungsreaktionen einhergehen, gelöst.

Überraschenderweise wurde festgestellt, dass 6,7,8-Trihydroxy-2H-1-benzopyran-2-on (Verbindung II) über besonders vorteilhafte pharmakologische Eigenschaften verfügt. Neben potenten antioxidativen Wirkungen hemmt die Verbindung außerdem die Synthese von Leukotrienen und Prostaglandinen sowie der proinflammatorischen Zytokine IL-1β, TNF-α und IL-6. Die Verbindung II ist deshalb grundsätzlich geeignet zur Behandlung oder Prophylaxe von Krankheiten, die mit oxidativem Stress einhergehen wie z. B. Diabetes Mellitus Typ I und/oder II, Atherosklerose und endotheliale Dysfunktion, Ischämien, neurologischen Erkrankungen (z. B. Alzheimersche Krankheit, Morbus Parkinson und andere neurodegerative Erkrankungen), Katarakt und Tumorerkrankungen. Verbindung II ist aber besonders vorteilhaft für Krankheitszustände mit einer entzündlichen Komponente wie z. B. rheumatoide Arthritis, Asthma, Colitis ulzerosa, Crohnsche Krankheit, Psoriasis, Neurodermitis, sowie Infektionen durch Bakterien, Viren (z. B. Influenza, AIDS, virale Hepatitis) und andere Erreger (z. B. Parasiten, Pilze und Prionen). Die Verbindung II wurde zwar bereits in der Literatur beschrieben (O. Kayser und H. Kolodziej, Phytochemistry 39, 1181 - 1185 (1995); S. Kumar, A. B. Ray, C. Konno, Y. Oshima und H. Hikino, Phytochemistry 27, 636 - 638 (1988); K. P. Latte, O. Kayser, N. Tan, M. Kaloga und H. Kolodziej, Z. Naturforsch. 55c, 528 - 533 (2000)), jedoch sind bisher keinerlei pharmakologische Effekte der Verbindung II bekannt. In Pelargonium sidoides ist Verbindung II in einer Konzentration von nur 0,0004 % (Kayser et al.; Latte et al., siehe oben) und in Pelargonium reniforme von nur 0,02 % enthalten (Latte et al., siehe oben), woraus zu schließen ist, dass Verbindung II in dieser niedrigen Konzentration keinen nennenswerten Beitrag zur biologischen Wirksamkeit von Pelargonium sidoides bzw. reniforme leistet.

Gegenstand der vorliegenden Erfindung ist deshalb die Verwendung der Verbindung II zur Behandlung oder Prophylaxe von Krankheitszuständen, die mit oxidativem Stress und/oder Entzündungsreaktionen einhergehen.

Es ist auch möglich, die Verbindung II in Form von Estern der Schwefelsäure der allgemeinen Formel I zu verabreichen, da Verbindung II aus diesen bei oraler Gabe freigesetzt wird. Aus diesem Grund sind auch die Verbindungen der allgemeinen Formel I zur Behandlung oder Prophylaxe der oben genannten Krankheitszustände geeignet. Bevorzugte Verbindungen der allgemeinen Formel I sind 6,7-Dihydroxy-8-sulfooxy-2H-1-benzopyran-2-on (R⁶ = R⁷ = H; R⁸ = SO₃H) und 7,8-Dihydroxy-6-sulfooxy-2H-1-benzopyran-2-on (R⁷ = R⁸ = H; R⁶ = SO₃H). Besonders bevorzugt ist 6,8-Bis(sulfooxy)-7-hydroxy-2H-1-benzopyran-2-on (R⁶ = R⁸ = SO₃H; R⁷ = H; Verbindung III). Die Verbindungen der allgemeinen Formel I, bei denen mindestens einer der Reste R⁶, R⁷ oder R⁸ ein SO₃H-Rest ist, sind neu. Diese und besonders die Verbindung III, sowie deren Verwendung zur Behandlung oder Prophylaxe von Krankheitszuständen, die mit oxidativem Stress und/oder Entzündungsreaktionen einhergehen sind deshalb ebenfalls Bestandteil der vorliegenden Erfindung.

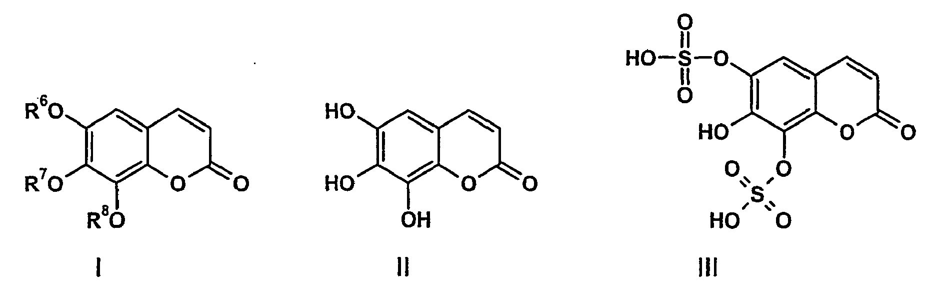

In der allgemeinen Formel I sind die Reste R⁶, R⁷ und R⁸ unabhängig voneinander ein Wasserstoffatom oder ein SO₃H-Rest. Die Verbindungen der allgemeinen Formel I, sowie die Verbindungen II und III können auch in Form von physiologisch verträglichen Alkali-, Erdalkali- oder anderen Salzen, z. B. von Kaliumsalzen vorliegen. Diese Salze sind ebenfalls Gegenstand der vorliegenden Erfindung.

Bestandteil der Erfindung sind auch Pflanzenextrakte, insbesondere aus Pelargonium-Arten, die eine oder mehrere der Verbindungen der allgemeinen Formel I, bei der mindestens einer der Reste R⁶, R⁷ und R⁸ ein SO₃H-Rest ist, enthalten, sowie daraus hergestellte pharmazeutische Zubereitungen. Bevorzugt sind dabei solche Extrakte, bei denen die Konzentration mindestens einer der Verbindung(en) der allgemeinen Formel I im Trockenanteil des Pflanzenextraktes zwischen 0,1 % und 10 %, besonders bevorzugt zwischen 0,5 % und 5 % liegt. Der Trockenanteil entspricht dabei dem Trockenrückstand gemäß Ph. Eur. (Fluidextrakte), wobei die Analyse auch direkt z. B. im Fluidextrakt erfolgen kann und der Trockenrückstand rechnerisch berücksichtigt wird.

Die Herstellung der Verbindung II kann durch Hydrolyse und/oder Etherspaltung z. B. aus kommerziell erhältlichem Fraxin oder aus einer Verbindung der allgemeinen Formel I, bei der mindestens einer der Reste R⁶, R⁷ und R⁸ ein SO₃H-Rest ist, erfolgen.

Die Herstellung derjenigen Verbindungen der allgemeinen Formel I, bei der mindestens einer der Reste R⁶, R⁷ und R⁸ ein SO₃H-Rest ist, kann durch Reaktion von Verbindung II mit Schwefeltrioxid-Trimethylamin-Komplex oder, im Falle der Verbindung III, durch Isolierung aus geeignetem Pflanzenmaterial, z. B. aus getrockneten Wurzeln von Pelargonium sidoides erfolgen. Die Verbindungen 6,7-Dihydroxy-8-sulfooxy-2H-1-benzopyran-2-on (allgemeine Formel I; R⁶ = R⁷ = H; R⁸ = SO₃H) und 7,8-Dihydroxy-6-sulfooxy-2H-1-benzopyran-2-on (allgemeine Formel I; R⁷ = R⁸ = H; R⁶ = SO₃H) können auch durch partielle Hydrolyse von Verbindung III erhalten werden.

Die erfindungsgemäßen Extrakte aus Pelargonien oder deren Pflanzenteilen können nach bekannten Herstellungsverfahren in variabler Zusammensetzung mit Lösungsmitteln wie z. B. Wasser, Methanol, Ethanol, Aceton, etc., und deren Gemische bei Temperaturen von Raumtemperatur bis 60 °C unter gelinder bis heftiger Durchmischung oder durch Perkolation innerhalb von 10 Min. bis 24 Std. erhalten werden. Bevorzugte Extraktionslösungsmittel sind dabei Wasser oder Gemische von Ethanol und Wasser mit mindestens 50 Gew.-% Wasseranteil, besonders bevorzugt im Verhältnis Ethanol / Wasser = 10/90 bis 15/85 (w/w). Zur Anreicherung der erfindungsgemäßen Verbindungen der allgemeinen Formel I können weitere Konzentrierungsschritte durchgeführt werden wie z. B. flüssig-flüssig-Verteilung mit z. B. 1-Butanol/Wasser oder Ethylacetat/Wasser, Adsorption-Desorption an lonenaustauscher, LH20, HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und / oder reduziertem Druck oder durch Gefriertrocknung. Trockenextrakte haben dabei gemäß dem Europäischen Arzneibuch im allgemeinen einen Trockenrückstand von mindestens 95 Gew.-%.

Die erfindungsgemäßen Verbindungen der allgemeinen Formel I bzw. die mindestens eine dieser Verbindungen enthaltenden Extrakte können in Form von Pulvern, Granulaten, Tabletten, Dragees oder Kapseln oder auch als Lösung vorzugsweise oral verabreicht werden.

Die Dosierung erfolgt dabei so, dass pro Tag 0,1 mg bis 250 mg, vorzugsweise 0,3 mg bis 50 mg einer oder mehrerer der Verbindungen der allgemeinen Formel I zugeführt werden.

Zur Herstellung von Tabletten wird mindestens eine der Verbindungen der allgemeinen Formel I bzw. der entsprechende Extrakt mit geeigneten pharmazeutisch verträglichen Hilfsstoffen wie z. B. Laktose, Cellulose, Siliciumdioxid, Croscarmellose und Magnesiumstearat gemischt und zu Tabletten gepresst, die gegebenenfalls mit einem geeigneten Überzug z. B. aus Hydroxymethylpropylcellulose, Polyethylenglykol, Farbstoffen (z. B. Titandioxid, Eisenoxid) und Talkum versehen werden.

Die Wirksamkeit von Verbindung II bei Krankheitszuständen, die mit oxidativem Stress und/oder Entzündungsreaktionen einhergehen, wird durch die nachstehend beschriebenen Versuche belegt.

### Antioxidative Eigenschaften:

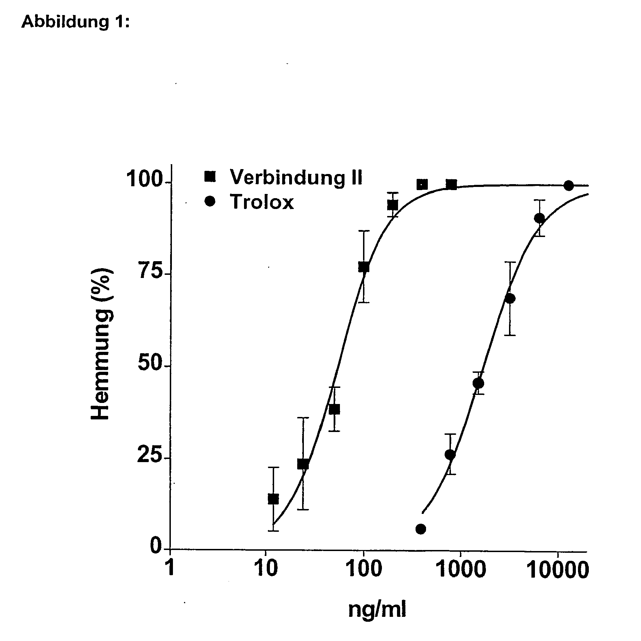

Die Autoxidation von Lipiden ist mit der Emission von Licht assoziiert. Die Bestimmung dieser ultraschwachen Chemilumineszenz kann sowohl zur Quantifizierung von Peroxiden als auch zur Beurteilung der Wirksamkeit von Antioxidantien verwendet werden. Als lipidreiches Gewebe diente in den vorliegenden Untersuchungen Hirngewebe männlicher Mäuse (NMRI; 20 - 30 g; Centre d'Elevage Janvier, Le Genest-Saint Isle, Frankreich). Nach der Entnahme wurde das Gehirn mit eiskalter phosphatgepufferter physiologischer Kochsalzlösung (PBS, pH 7,4) gewaschen und von Meningen sowie Blutresten befreit wurde. Die Gewebeproben wurden in einem 4-fachen Volumen (v/w) PBS homogenisiert und 10 Minuten lang bei 1000 x g und 4 °C zentrifugiert. Die Überstände wurden sofort mit demselben Puffer auf das 3-fache ihres Volumens verdünnt und auf Eis aufbewahrt. 250 µl des dilutierten Überstandes wurden in ein Teströhrchen überführt und 10 Minuten lang bei 37 °C in einem 6-Kanal-Luminometer (Multi-Biolumat LB 9505 C, Berthold, Bad Wildbad) inkubiert. Nach Zufügen von 25 µl der Verbindung II in PBS mit 2,5 % DMSO wurde die Inkubation für weitere 10 Minuten fortgesetzt. Danach wurde die Intensität der Chemilumineszenz (CL) für die Dauer von 60 Minuten bestimmt. Die prozentuale Hemmung der Autoxidation wurde im Vergleich zu einer gleichzeitig gemessenen Lösungsmittelkontrolle (PBS mit 2,5 % DMSO) berechnet. Die Verbindung II hemmte die Autoxidation der Lipide äußerst potent mit einer halbmaximalen Hemmkonzentration von 53 ng/ml (Abbildung 1). Die bei Bestimmungen antioxidativer Eigenschaften häufig verwendete Vergleichssubstanz Trolox zeigte dagegen nur eine halbmaximale Hemmkonzentration von 1665 ng/ml.

Abbildung 1 zeigt den Einfluß von Verbindung II und Trolox auf die Autoxidation von Lipiden. Angegeben ist die prozentuale Hemmung der Lipidperoxidation im Vergleich zu einer Lösungsmittelkontrolle aus drei unabhängigen Versuchen (Mittelwert ± SD).

### Hemmung der Synthese von proinflammatorischen Zytokinen:

Der Einfluß von Verbindung II auf die Synthese der proinflammatorischen Zytokine IL-1β, TNF-α und IL-6 wurde unter Verwendung von aktivierten murinen Peritoneal-Makrophagen bestimmt. Für die Gewinnung der aktivierten Makrophagen wurden männlichen NMRI-Mäusen (Centre d'Elevage Janvier, Le Genest-Saint lsle, Frankreich) 3×10⁹ abgetötete Corynbakterium parvum-Bakterien (Changzhou Yanshen Co. Ltd., Changzhou, China) in 0,5 ml PBS intraperitoneal injiziiert. Die Bauchhöhle wurde 6 Tage später mit 2,5 ml Hanks-bilanzierter Salzlösung (HBSS) ohne Calcium und Magnesium unter Zusatz von 10 U/ml Heparin gespült. Die Zellen wurde in einer Konzentration von 2×10⁶ Zellen/ml in kompletten RPMI-Medium mit Zusatz von 10% fötalem Kälberserum resuspendiert. Jeweils 200 µl Zellsuspension wurde in die Vertiefungen von 96-Well Mikrotiterplatten eingefüllt. Nach einer Inkubationsphase von 2 h wurden nicht adhärente Zelllen entfernt und der verbleibende Zellrasen zweimal mit Kulturmedium (37 °C) gewaschen. Die Makrophagen wurden für 30 min mit der Verbindung II vorinkubiert und die Synthese von proinflammatorischen Zytokinen dann durch Zugabe von 1 µg/ml Lipopolysaccharid von E. coli (Serotyp 0127:B8, Sigma, Deisenhofen) induziert. Nach einer Inkubation für 24 h (37 °C, 5 % CO₂ in Luft) wurden die Zellen durch dreimaliges Einfrieren und Auftauen lysiert, die Zellüberstände gewonnen und bis zur Analyse bei -80 °C eingefroren. Die Bestimmung der Zytokinkonzentration in den Zellüberständen erfolgte mit Hilfe von kommerziellen Testkits (Duosets IL-1β, TNF-α und IL-6, R&D, Wiesbaden) entsprechend den Vorschriften des Herstellers. Alle Untersuchungen erfolgten in Dreifachbestimmung. Der Einfluß der Verbindung II auf die Synthese der Zytokine wurde im Vergleich zu parallel getesteten Lösungsmittelkontrollen (0,1 % DMSO in kompletten RPMI-Medium) beurteilt. Wie aus der nachstehenden Tabelle 1 ersichtlich ist, bewirkte die Verbindung II bei einer Konzentration von 100 µg/ml eine signifikante Hemmung der Synthese aller drei gemessenen Zytokine, wobei die Wirkung auf die Produktion von IL-6 am stärksten ausgeprägt war.

**Tabelle 1: Einfluss von Verbindung II auf die Synthese von proinflammatorischen Zytokinen in aktivierten Maus-Peritonal-Makrophagen. Angegeben sind die Mittelwerte ± SD aus drei parallelen Testansätzen. Der Effekt von Verbindung II wurde kalkuliert als prozentuale Veränderung im Vergleich zu einer Lösungsmittelkontrolle (* Irrtumswahrscheinlichkeit P < 0.05, t-Test).**

| Testansatz | IL-1β | | TNF-α | | IL-6 | |
|---|---|---|---|---|---|---|
| | pg/ml | Effekt (%) | pg/ml | Effekt (%) | pg/ml | Effekt (%) |
| Kontrolle | 3553 ± 293 | | 8393 ± 923 | | 31449 ± 2201 | |
| Verbindung II 100 µg/ml | 430 ± 65 | -88* | 6251 ± 7 | -26* | 976 ± 244 | -97* |
| Verbindung II 30 µg/ml | 3356 ± 87 | -6 | 10189 ± 1018 | +21 | 22519 ± 3153 | -28* |
| Verbindung II 10 µg/ml | 3789 ± 72 | +7 | 10050 ± 462 | +19 | 23078 ± 3461 | -27* |

### Hemmung von Cyclooxygenase- und Lipoxygenase-Aktivität in humanem Vollblut:

Für die Untersuchungen wurde heparinisiertes humanes Vollblut verwendet. Pro Vertiefung wurde 100 µl Vollblut in 96-Well Mikrotiterplatten vorgelegt. Getrennte Platten wurden zur Bestimmung der Cyclooxygenase-1 (COX1)- und Lipoxygenase (LO)-Aktivität sowie zur Induktion von Cyclooxygenase-2 (COX2) verwendet.

Verbindung II wurde in DME-Medium (DMEM) mit 1 % Antibiotika/Antimykotika-Lösung und 2 mM L-Glutamin (Sigma, Deisenhofen) unter Verwendung von DMSO (Endkonzentration 0,1 %) als Lösungsvermittler verdünnt. Nach Zugabe von 50 µl Verbindung II wurden die Testansätze vor 60 min bei 37 °C inkubiert. Anschließend wurde zur Stimulation der Eicosanoid-Synthese 50 µl Calciumionophor A23187 (Endkonzentration 50 µM) zugefügt. Nach weiteren 30 min Inkubation bei 37 °C wurden die Mikrotiterplatten für 5 min bei 4 °C mit 1500 g zentrifugiert. Das Plasma wurde abpipettiert und bis zur Analyse bei -80 °C eingefroren.

Für den Nachweis von COX2-Aktivität wurden die Blutproben (100 µl/Vertiefung) zunächst zur Inaktivierung der COX1 für 6 h bei 37 °C mit Aspirin (50 µl in DMEM, Endkonzentration 12 µg/ml) vorbehandelt. Anschließend wurde die Verbindung II bzw. das Lösungsmittel (DMEM mit 0,1% DMSO) in einem Volumen von 25 µl zugefügt. Zur Induktion der Expression von COX2 wurde außerdem 25 µl Lipopolysaccharid von E. coli (Serotyp 0127:B8, Endkonzentration 10 µg/ml) zugegeben. Nach Inkubation für 18 h bei 37 °C wurde das Plasma wie oben beschrieben gewonnen und ebenfalls bis zur Analyse bei -80 °C aufbewahrt.

In den Plasmaproben wurden TXB₂, PGE₂ und Cystenyl-Leukotriene (Cystenyl-LT) als Parameter der COX1-, COX2- und LO-Aktivität bestimmt. Für die Analyse wurden kommerzielle EIA-Testkits (TXB2 und PGE2: Caymann/IBL, Hamburg; Cystenyl-Leukotriene: CAST-2000, Milenia, Bad Nauheim) entsprechend den Herstellervorschriften verwendet. Aus den Ergebnissen (siehe Tabelle 2) wird deutlich, dass Verbindung II eine potente Hemmung der Aktivität von COX2 und LO bewirkt. Die Aktivität von COX1 wird hingegen kaum beeinflußt. Dieses Wirkspektrum ist als äußerst vorteilhaft anzusehen, da bei der therapeutischer Anwendung von Verbindung II deshalb nicht mit den für COX1-Inhibitoren typischen Nebenwirkungen, wie z. B. gastrointestinalen Komplikationen (Erosionen, Ulzerationen) oder Blutungen durch Hemmung der Thrombozytenaggregation gerechnet werden muss.

**Tabelle 2: Einfluß von Verbindung II auf die Synthese von Cystenyl-LT, TXB₂ und PGE₂ in humanem Vollblut. Angegeben sind die Mittelwerte ± SD aus zwei parallelen Testansätzen. Der Effekt von Verbindung II wurde kalkuliert als prozentuale Veränderung im Vergleich zu einer Lösungsmittelkontrolle.**

| Testansatz | Cystenyl-LT | | TXB₂ | | PGE₂ | |
|---|---|---|---|---|---|---|
| | pg/ml | Effekt (%) | pg/ml | Effekt (%) | pg/ml | Effekt (%) |
| Kontrolle | 7227 ± 612 | | 9777 ± 1389 | | 10773 ± 944 | |
| Verbindung II 100 µg/ml | 661 ± 520 | -91 | 9115 ± 244 | -7 | 2232 ± 68 | -79 |
| Verbindung II 30 µg/ml | 2089 ± 90 | -71 | 9993 ± 6658 | +2 | 2626 ± 503 | -76 |
| Verbindung II 10 µg/ml | 3557 ± 86 | -51 | 7825 ± 881 | -20 | 3679 ± 41 | -66 |
| Verbindung II 3 µg/ml | 5193 ± 542 | -28 | 7278 ± 430 | -26 | 5499 ± 86 | -49 |

### Beispiel 1: Herstellung von 6,7,8-Trihydroxy-2H-1-benzopyran-2-on (Verbindung II)

20 g (42.7 mmol) 6,8-Bis(sulfooxy)-7-hydroxy-2H-1-benzopyran-2-on Kaliumsalz wurden in 480 ml ca. 2 N Salzsäure 20 h bei 40 bis 50 °C gerührt. Nach dem Abkühlen wurde das ausgefallene Rohprodukt abfiltriert und aus Wasser umkristallisiert (Heißfiltration). Das Kristallisat wurde abgesaugt, gewaschen und bei 100 °C im Vakuum getrocknet: 5.9 g (71 %), Schmelzpunkt: Zersetzung ab 260 °C; ¹H- und ¹³C-NMR stimmen mit den Angaben von O. Kayser und H. Kolodziej (Phytochemistry 39, 1181 -1185 (1995)) überein.

### Beispiel 2: Isolierung und Strukturaufklärung von 6,8-Bis(sulfooxy)-7-hydroxy-2H-1-benzopyran-2-on Kaliumsalz (Kaliumsalz von Verbindung III)

15 kg gemahlene Wurzeln von Pelargonium sidoides wurden bei Raumtemperatur zweimal mit 75 L bzw. 40 L Wasser perkoliert. Der wässrige Extrakt wurde auf etwa 1/3 eingeengt, mit 7 kg Ammoniumsulfat versetzt und mehrmals mit einem 2-Butanon / Ethanol 3/2 Gemisch extrahiert. Die organischen Phasen wurden vereinigt und eingedampft.

Dieser Rückstand wurde über eine HP20 Säule chromatographiert (Eluens: Wasser). Die 6,8-Bis(sulfooxy)-7-hydroxy-2H-1-benzopyran-2-on-Fraktionen wurden aufkonzentriert, mit Kalilauge auf pH 8 eingestellt und mit Ethanol im Verhältnis 1/1 verdünnt. Der ausgefallene Niederschlag wurde abfiltriert und in Wasser suspendiert. Mit Kalilauge wurde auf pH 10.7 eingestellt und mit Ethanol im Verhältnis 1/1 verdünnt. Der daraufhin ausgefallene Niederschlag wurde wiederum in heißem Wasser gelöst. Die heiße Lösung wurde filtriert und mit Ethanol im Verhältnis 1/1 verdünnt. Das ausfallende Kristallisat wurde abgesaugt, gewaschen und bei 50 °C im Vakuum getrocknet: 27.6 g (0.14 % bezogen auf das Pflanzenmaterial, berechnet als freie Säure).

Schmelzpunkt: Zersetzung ab 216 °C; C₉H₃K₃O₁₁S₂ (468.55) gefunden: C 23.08 %, H 0.70 %, K 24.65 %, S 13.9 % - berechnet: C 23.07 %, H 0.65 %, K 25.04 %, O 37.56 %, S 13.69 %; ¹H-NMR (DMSO-d₆): δ = 7.62 (d, J = 9.1 Hz, H-4), 7.03 (s, H-5), 5.59 (d, J = 9.1 Hz, H-3); ¹³C-NMR (DMSO-d₆): δ = 162.9 (C-7), 161.9 (C-2), 147.7 (C-8), 145.1 (C-4), 142.2 (C-6), 130.4 (C-8a), 115.5 (C-5), 102.4 (C-3), 101.5 (C-4a).

Die saure Hydrolyse von Trihydroxycumarin-Disulfat Kaliumsalz führt zu 6,7,8-Trihydroxycumarin (siehe Beispiel 1). Zur weiteren Aufklärung der Struktur wurde die Verbindung III gemäß nachstehender Reaktionsfolge derivatisiert:

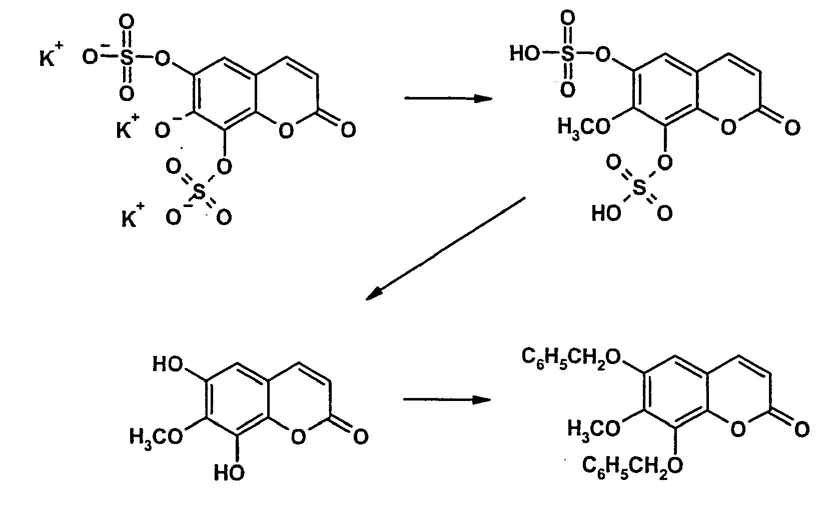

Dazu wurde das Trihydroxycumarin-Disulfat Kaliumsalz mit lodmethan in Gegenwart von Kaliumcarbonat bei 60 °C in DMF zum entsprechenden 7-Methylether umgesetzt. Nach Ansäuern mit konzentrierter Salzsäure wurde 24 h bei 50 °C gerührt, mit Ethylacetat extrahiert und über Kieselgel chromatografiert (Eluens: Heptan / Ethylacetat 7/3): 6,8-Dihydroxy-7-methoxycumarin. Dieses wurde in Gegenwart von Kaliumcarbonat und Kaliumiodid in DMF bei Raumtemperatur mit Benzylbromid umgesetzt. Die Mischung wurde eingeengt und der Rückstand zwischen Wasser und TBME verteilt. Die organische Phase wurde eingeengt und über Kieselgel chromatografiert (Eluens: Toluol / Ethanol 95/5): 6,8-Dibenzyloxy-7-methoxycumarin.

Das Substitutionsmuster letztgenannter Verbindung wurde mit ein- und zweidimensionaler NMR-Spektroskopie in CDCl₃ aufgeklärt. Eine deutliche NOESY-Korrelation zwischen H-5 und einem der CH₂-Signale lässt eindeutig auf einen Benzyloxyrest in 6-Position schließen. Ferner korreliert das OCH₃-Signal mit beiden CH₂-Signalen, woraus hervorgeht, dass die Methoxygruppe zwischen den beiden Benzyloxyresten, also in der 7-Stellung, positioniert ist. Die HMBC-Korrelationen zwischen C-7 und H-5 sowie OCH₃, sowie zwischen C-8 und H-4 sowie 8-CH₂ bestätigen das aus dem NOESY entnommene Substitutionsmuster. Aus dem Herstellungsweg des untersuchten Derivates und dessen Struktur lässt sich eindeutig ableiten, dass die Sulfoxyreste des Trihydroxycumarin-Disulfats in den Positionen 6 und 8 gebunden sind.

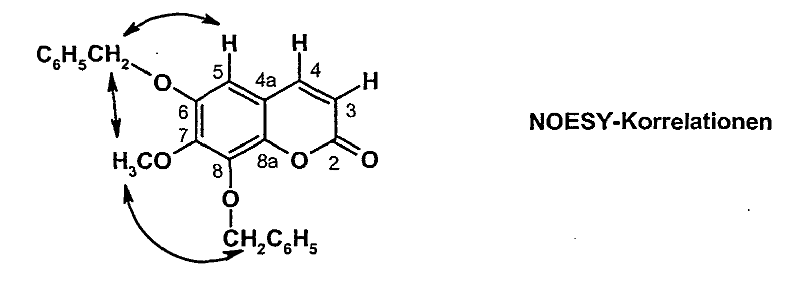

### Beispiel 3: Pflanzenextrakt mit einem Gehalt an 6,8-Bis(sulfooxy)-7-hydroxy-2H-1-benzopyran-2-on (Verbindung III)

500 g gemahlene Wurzeln aus Pelargonium sidoides wurden mit 3 kg Wasser 4 h bei Raumtemperatur extrahiert. Das ausextrahierte Pflanzenmaterial wurde abfiltriert und noch einmal mit 2 kg Wasser wie oben extrahiert und filtriert. Die Filtrate wurden vereinigt, bei ca. 35 °C aufkonzentriert und gefriergetrocknet: 58.7 g (11.7 %) Trockenextrakt mit einem Gehalt von 1.54 % der Verbindung III.

### Beispiel 4: Pflanzenextrakt mit einem Gehalt an 6,8-Bis(sulfooxy)-7-hydroxy-2H-1-benzopyran-2-on (Verbindung III)

Ca. 1.25 kg gemahlene Wurzeln aus Pelargonium sidoides wurden mit ca. 12.5 kg Ethanol / Wasser 11 / 89 (w/w) bei Raumtemperatur extrahiert. Nach Filtration wurde das Filtrat bei ca. 45 °C aufkonzentriert und gefriergetrocknet: 90.4 g (7.2 %) Trockenextrakt mit einem Gehalt von 1.86 % der Verbindung III.

### Beispiel 5: Tabletten

Zur Herstellung von Tabletten, die je nach gewünschter Wirkungsstärke 5 bis 250 mg Wirkstoff enthalten, benötigt man

| | |
|---|---|
| Verbindung II | 200 bis 5 000 g |
| Zellulosepulver | 2 000 g |
| Maisstärke | 1 200 g |
| kolloide Kieselsäure | 80 g |
| Magnesiumstearat | 20 g |
| Milchzucker | ad 10 000 g |

Der Wirkstoff wird gegebenenfalls gemahlen, mit den Hilfsstoffen homogen vermischt und in der üblichen Weise zu Tabletten von je 250 mg Gewicht und 9 mm Durchmesser verpresst. Bei Dosierungen über 125 mg presst man Tabletten von je 500 mg Gewicht und 11 mm Durchmesser. Falls gewünscht, werden die Tabletten mit einem Filmüberzug versehen.

## Patentansprüche

1. Verwendung von Verbindungen der allgemeinen Formel I, 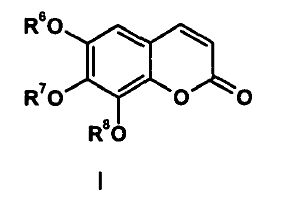 in der die Reste R⁶, R⁷ und R⁸ unabhängig voneinander H oder SO₃H bedeuten, sowie deren physiologisch verträgliche Salze zur Herstellung eines Arzneimittels zur Behandlung oder Prophylaxe bzw. eines diätetischen Nahrungsmittels zur Unterstützung der Behandlung oder zur Prophylaxe von Krankheitszuständen, die mit oxidativem Stress und/oder Entzündungsreaktionen einhergehen.

2. Verwendung nach Anspruch 1, wobei die Reste R⁶, R⁷ und R⁸ WasserstoffAtome sind.

3. Verwendung nach Anspruch 1, wobei die Reste R⁶ und R⁸ SO₃H und der Rest R⁷ Wasserstoff bedeuten.

4. Verwendung nach einem der Ansprüche 1 bis 3, wobei eine oder mehrere der Verbindungen der allgemeinen Formel I in einem Pflanzenextrakt enthalten sind.

5. Verwendung nach Anspruch 4, wobei der Pflanzenextrakt ein Extrakt aus Pelargonium-Arten ist.

6. Verwendung nach Anspruch 5, wobei der Pflanzenextrakt ein Extrakt aus Pelargonium sidoides ist.

7. Verwendung nach einem der Ansprüche 4 bis 6, wobei die Konzentration mindestens einer der Verbindung(en) der allgemeinen Formel I im Trockenanteil des Pflanzenextraktes zwischen 0,1 % und 10 % liegt.

8. Verwendung nach einem der Ansprüche 4 bis 6, wobei die Konzentration mindestens einer der Verbindung(en) der allgemeinen Formel I im Trockenanteil des Pflanzenextraktes zwischen 0,5 % und 5 % liegt.

9. Verwendung nach einem der Ansprüche 1 bis 8, wobei der Krankheitszustand ausgewählt ist aus der Gruppe umfassend Diabetes Mellitus Typ I und/oder II, entzündliche Erkrankungen umfassend rheumatoide Arthritis, Asthma, Colitis Ulzerosa, Crohnsche Krankheit, Psoriasis, Neurodermitis,
Infektionen durch Bakterien, Viren umfassend Influenza, AIDS, virale Hepatitis und andere Erreger umfassend Parasiten, Pilze und Prionen,
Artherosklerose und endotheliale Dysfunktion,
lschämien,
neurologischen Erkrankungen umfassend Alzheimersche Krankheit, Morbus Parkinson und andere neurodegerative Erkrankungen oder
Katarakt und Tumorerkrankungen.

10. Verbindung der allgemeinen Formel I, 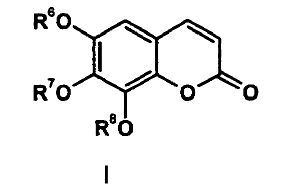 in der die Reste R⁶, R⁷ und R⁸ unabhängig voneinander H oder SO₃H bedeuten, sowie deren physiologisch verträgliche Salze,
mit der Maßgabe, dass R⁶, R⁷ und R⁸ nicht gleichzeitig H bedeuten.

11. Verbindungen nach Anspruch 10, in der
R⁶ und R⁸ SO₃H und R⁷ Wasserstoff bedeuten, oder
R⁶ SO₃H und R⁷ und R⁸ Wasserstoff bedeuten, oder
R⁸ SO₃H und R⁶ und R⁷ Wasserstoff bedeuten.

12. Pflanzenextrakt, enthaltend eine oder mehrere der Verbindungen nach Anspruch 10 oder 11.

13. Pflanzenextrakt nach Anspruch 12, wobei der Pflanzenextrakt ein Extrakt aus Pelargonium-Arten ist.

14. Pflanzenextrakt nach Anspruch 13, wobei der Pflanzenextrakt ein Extrakt aus Pelargonium sidoides ist.

15. Pflanzenextrakt nach einem der Ansprüche 12 bis 14, wobei die Konzentration mindestens einer der Verbindung(en) nach Anspruch 10 oder 11 im Trockenanteil des Pflanzenextraktes zwischen 0,1 % und 10 % liegt.

16. Pflanzenextrakt nach Anspruch 15, wobei die Konzentration mindestens einer der Verbindung(en) nach Anspruch 10 oder 11 im Trockenanteil des Pflanzenextraktes zwischen 0,5 % und 5 % liegt.

17. Arzneimittel zur Behandlung oder Prophylaxe von Krankheitszuständen, die mit oxidativem Stress und/oder Entzündungsreaktionen einhergehen mit einem Gehalt an mindestens einer der Verbindungen der allgemeinen Formel I wie in Anspruch 1, 2 oder 3 definiert.

18. Diätetisches Nahrungsmittel zur Unterstützung der Behandlung oder zur Prophylaxe von Krankheitszuständen, die mit oxidativem Stress und/oder Entzündungsreaktionen einhergehen mit einem Gehalt an mindestens einer der Verbindungen der allgemeinen Formel I wie in Anspruch 1, 2 oder 3 definiert.

19. Pharmazeutische Zubereitung, bestehend aus mindestens einer der Verbindungen der allgemeinen Formel I, wie in Anspruch 1, 2 oder 3 definiert, und geeigneten Hilfsstoffen als orale Darreichungsform.

20. Pharmazeutische Zubereitung, bestehend aus einem Pflanzenextrakt nach einem der Ansprüche 12 bis 16 und geeigneten Hilfsstoffen als orale Darreichungsform.

## Claims

1. Use of the compounds of the general formula I, 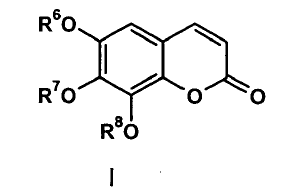 wherein the residues R⁶, R⁷ and R⁸ independently represent H or SO₃H, and the physiologically acceptable salts thereof for the manufacture of a medicament for the treatment or prophylaxis, or a dietetic food product for supporting the treatment, or for the prophylaxis of pathological conditions associated with oxidative stress and/or inflammatory reactions.

2. Use according to claim 1, wherein the residues R⁶, R⁷ and R⁸ are hydrogen atoms.

3. Use according to claim 1, wherein the residues R⁶ and R⁸ represent SO₃H and the residue R⁷ represents hydrogen.

4. Use according to any one of claims 1 to 3, wherein one or more of the compounds of general formula I are contained in a plant extract.

5. Use according to claim 4, wherein the plant extract is an extract from Pelargonium species.

6. Use according to claim 5, wherein the plant extract is an extract from Pelargonium sidoides.

7. Use according to any one of claims 4 to 6, wherein the concentration of at least one of the compound(s) of general formula I in the dry content of the plant extract is between 0.1 % and 10 %.

8. Use according to any one of claims 4 to 6, wherein the concentration of at least one of the compound(s) of general formula I in the dry content of the plant extract is between 0.5 % and 5 %.

9. Use according to any one of claims 1 to 8, wherein the pathological condition is selected from the group comprising diabetes mellitus type I and/or II,
inflammatory diseases comprising rheumatoid arthritis, asthma, colitis ulcerosa, Morbus Crohn, psoriasis, neurodermitis,
infections by bacteria, viruses comprising influenza, AIDS, viral hepatitis and other pathogens comprising parasites, fungi and prions,
artherosclerosis and endothelial dysfunktion,
ischemia,
neurologic diseases comprising Morbus Alzheimer, Morbus Parkinson and other neurodegenerative diseases or
cataract and tumor diseases.

10. Compound of general formula I, 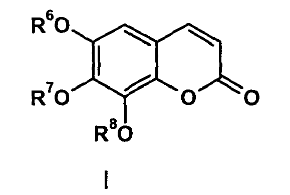 wherein the residues R⁵, R⁷ and R⁸ independently represent H or SO₃H and the physiologically acceptable salts thereof,
provided that R⁶, R⁷ and R⁸ are not H at the same time.

11. Compounds according to claim 10, wherein
R⁶ and R⁸ represent SO₃H and R⁷ represents hydrogen, or
R⁶ represents SO₃H and R⁷ and R⁸ represent hydrogen, or
R⁸ represents SO₃H and R⁶ and R⁷ represent hydrogen.

12. Plant extract, containing one or more of the compounds according to claim 10 or 11.

13. Plant extract according to claim 12, wherein the plant extract is an extract from Pelargonium species.

14. Plant extract according to claim 13, wherein the plant extract is an extract from Pelargonium sidoides.

15. Plant extract according to any one of claims 12 to 14, wherein the concentration of at least one of the compound(s) according to claim 10 or 11 in the dry content of the plant extract is between 0.1 % and 10 %.

16. Plant extract according to claim 15, wherein the concentration of at least one of the compound(s) according to claim 10 or 11 in the dry content of the plant extract is between 0.5 % and 5 %.

17. Medicament for the treatment or prophylaxis of pathological conditions associated with oxidative stress and/or inflammatory reactions having a content of at least one of the compounds of general formula I as defined in claim 1, 2 or 3.

18. Dietetic food product for supporting the treatment or for the prophylaxis of pathological conditions associated with oxidative stress and/or inflammatory reactions having a content of at least one of the compounds of general formula I as defined in claim 1, 2 or 3.

19. Pharmaceutical preparation consisting of at least one of the compounds of general formula I as defined in claim 1, 2 or 3, and suitable adjuvants as an oral administration form.

20. Pharmaceutical preparation consisting of a plant extract according to any one of claims 12 to 16 and suitable adjuvants as an oral administration form.

## Revendications

1. Utilisation de composés de formule générale I, 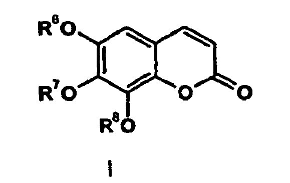 dans laquelle les radicaux R⁶, R⁷ et R⁸ représentent chacun indépendamment des autres H ou SO₃H, ainsi que de leurs sels acceptables d'un point de vue physiologique, pour la préparation d'un médicament destiné au traitement ou à la prophylaxie, ou d'un aliment diététique destiné à aider au traitement ou à la prophylaxie d'états pathologiques qui s'accompagnent d'un stress oxydatif et/ou de réactions inflammatoires.

2. Utilisation selon la revendication 1, pour laquelle les radicaux R⁶, R⁷ et R⁸ sont des atomes d'hydrogène.

3. Utilisation selon la revendication 1, pour laquelle les radicaux R⁶ et R⁸ sont SO₃H, et le radical R⁷ est un atome d'hydrogène.

4. Utilisation selon l'une des revendications 1 à 3, pour laquelle un ou plusieurs des composés de formule générale I sont contenus dans un extrait végétal.

5. Utilisation selon la revendication 4, pour laquelle l'extrait végétal est un extrait d'espèces de pélargonium.

6. Utilisation selon la revendication 5, pour laquelle l'extrait végétal est un extrait de Pelargonium sidoides.

7. Utilisation selon l'une des revendications 4 à 6, pour laquelle la concentration d'au moins l'un des composés de formule générale I dans la fraction sèche de l'extrait végétal est comprise entre 0,1 % et 10 %.

8. Utilisation selon l'une des revendications 4 à 6, pour laquelle la concentration d'au moins l'un des composés de formule générale I dans la fraction sèche de l'extrait végétal est comprise entre 0,5 % et 5 %.

9. Utilisation selon l'une des revendications 1 à 8, pour laquelle l'état pathologique est choisi dans le groupe comprenant le diabète sucré de type I et/ou de type II, les maladies inflammatoires comprenant la polyarthrite rhumatoïde, l'asthme, la recto-colite hémorragique, la maladie de Crohn, le psoriasis, la névrodermite, des infections par des bactéries, des virus parmi lesquels la grippe, le SIDA, l'hépatite virale et d'autres agents pathogènes comprenant des parasites, des champignons et des prions, l'athérosclérose et le dysfonctionnement endothélial, les ischémies, les maladies neurologiques, parmi lesquelles la maladie d'Alzheimer, la maladie de Parkinson et d'autres maladies neurodégénératives, ou la cataracte et les maladies tumorales.

10. Composé de formule générale 1 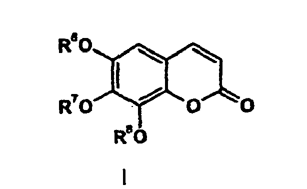 dans laquelle les radicaux R⁶, R⁷ et R⁸ représentent chacun indépendamment des autres H ou SO₃H, ainsi que leurs sels acceptables d'un point de vue physiologique, à la condition que R⁶, R⁷ et R⁸ ne représentent pas simultanément H.

11. Composés selon la revendication 10, dans laquelle
R⁶ et R⁸ sont SO₃H et R⁷ est un atome d'hydrogène, ou
R⁶ est SO₃H et R⁷ et R⁸ sont des atomes d'hydrogène, ou
R⁸ est SO₃H et R⁶ et R⁷ sont des atomes d'hydrogène.

12. Extrait végétal contenant un ou plusieurs des composés selon la revendication 10 ou II.

13. Extrait végétal selon la revendication 12, cet extrait végétal étant un extrait d'espèces de pélargonium.

14. Extrait végétal selon la revendication 13, cet extrait végétal étant un extrait de Pelargonium sidoides.

15. Extrait végétal selon l'une des revendications 12 à 14, dans lequel la concentration d'au moins un des composés selon la revendication 10 ou 11 dans la fraction sèche de l'extrait végétal est comprise entre 0,1 % et 10 %.

16. Extrait végétal selon la revendication 15, dans lequel la concentration d'au moins un des composés selon la revendication 10 ou 11 dans la fraction sèche de l'extrait végétal est comprise entre 0,5 % et 5 %.

17. Médicament destiné au traitement ou à la prophylaxie d'états pathologiques qui s'accompagnent d'un stress oxydatif et/ou de réactions inflammatoires, ayant une certaine teneur en au moins un des composés de formule générale I tels que définis dans la revendication 1, 2 ou 3.

18. Aliment diététique destiné à aider au traitement et/ou à la prophylaxie d'états pathologiques qui s'accompagnent d'un stress oxydatif et/ou de réactions inflammatoires, ayant une certaine teneur en au moins un des composés de formule générale I tels que définis dans la revendication 1, 2 ou 3.

19. Préparation pharmaceutique constituée d'au moins un des composés de formule générale I tels que définis dans la revendication 1, 2 ou 3, et d'adjuvants appropriés, en tant que forme posologique orale.

20. Préparation pharmaceutique constituée d'un extrait végétal selon l'une des revendications 12 à 16 et d'adjuvants appropriés, en tant que forme posologique orale.
